# EUROPEAN PATENT SPECIFICATION

(11) **EP 2 091 551 B1**
(45) Date of publication and mention of the grant of the patent: **15.01.2020**
(21) Application number: 07871367.4
(22) Date of filing: 05.11.2007
(51) Int. Cl.: A61K 38/00, A61K 38/04, A61K 38/08, G01N 33/53, G01N 33/567

(54) **METHOD OF TREATING ASTHMA**
VERFAHREN ZUR BEHANDLUNG VON ASTHMA
PROCEDE DE TRAITEMENT DE L'ASTHME

(30) Priority: 03.11.2006 US 856380 P
(43) Date of publication of application: 26.08.2009
(73) Proprietor: Alba Therapeutics Corporation, Baltimore, MD 21201 (US)
(72) Inventor: PATERSON, Blake, Baltimore, MD 21231 (US)
(74) Representative: Mewburn Ellis LLP
(86) International application number: PCT/US2007/083633
(87) International publication number: WO 2008/073627

(56) References cited:
- US-A1- 2005 019 314
- US-A1- 2005 059 593
- US-A1- 2005 059 593
- REED C E ET AL: "The role of protease activation of inflammation in allergic respiratory diseases" JOURNAL OF ALLERGY AND CLINICAL IMMUNOLOGY, MOSBY, INC, US, vol. 114, no. 5, 1 November 2004 (2004-11-01), pages 997-1008, XP004631188 ISSN: 0091-6749
- D. RITTIRSCH ET AL: "Zonulin as prehaptoglobin2 regulates lung permeability and activates the complement system", AMERICAN JOURNAL OF PHYSIOLOGY-LUNG CELLULAR AND MOLECULAR PHYSIOLOGY, vol. 304, no. 12, 15 June 2013 (2013-06-15), pages 863-872, XP055309866, ISSN: 1040-0605, DOI: 10.1152/ajplung.00196.2012
- Atsufumi Kawabata: "PAR-2: structure, function and relevance to human diseases of the gastric mucosa expert reviews in molecular medicine PAR-2: structure, function and relevance to human diseases of the gastric mucosa", Expert Reviews in Molecular Medicine, vol. 4, no. 16, 1 July 2002 (2002-07-01), pages 1-17, XP055310201,
- A Fasano ET AL: "ZOT/ZONULIN-MEDIATED REGULATION OF INTESTINAL TIGHT JUNCTIONS INVOLVES THE PROTEINASE-ACTIVATED RECEPTOR 2 (PAR-2)", Journal of Pediatric Gastroenterology and Nutrition, vol. 39, 1 June 2004 (2004-06-01), pages S253-S254, XP055492279,

### SEQUENCE LISTING

<110> Alba Therapeutics Corporation
<120> METHODS OF DIAGNOSING AND TREATING ASTHMA
<130> 22344-00002-WO
<150> 60/856,380
   <151> 2006-11-03
<160> 24
<170> PatentIn version 3.4
<210> 1
   <211> 8
   <212> PRT
   <213> Artificial
<220>
   <223> chemically synthesized
<400> 1
   Gly Arg Val Cys Val Gln Pro Gly
   1               5
<210> 2
   <211> 8
   <212> PRT
   <213> Artificial
<220>
   <223> chemically synthesized
<400> 2
   Gly Arg Val Cys Val Gln Asp Gly
   1               5
<210> 3
   <211> 8
   <212> PRT
   <213> Artificial
<220>
   <223> chemically synthesized
<400> 3
   Gly Arg Val Leu Val Gln Pro Gly
   1               5
<210> 4
   <211> 8
   <212> PRT
   <213> Artificial
<220>
   <223> chemically synthesized
<400> 4
   Gly Arg Val Leu Val Gln Asp Gly
   1               5
<210> 5
   <211> 8
   <212> PRT
   <213> Artificial
<220>
   <223> chemically synthesized
<400> 5
   Gly Arg Leu Cys Val Gln Pro Gly
   1               5
<210> 6
   <211> 8
   <212> PRT
   <213> Artificial
<220>
   <223> chemically synthesized
<400> 6
   Gly Arg Leu Cys Val Gln Asp Gly
   1               5
<210> 7
   <211> 8
   <212> PRT
   <213> Artificial
<220>
   <223> chemically synthesized
<400> 7
   Gly Arg Leu Leu Val Gln Pro Gly
   1               5
<210> 8
   <211> 8
   <212> PRT
   <213> Artificial
<220>
   <223> chemically synthesized
<400> 8
   Gly Arg Leu Leu Val Gln Asp Gly
   1               5
<210> 9
   <211> 8
   <212> PRT
   <213> Artificial
<220>
   <223> chemically synthesized
<400> 9
   Gly Arg Gly Cys Val Gln Pro Gly
   1               5
<210> 10
   <211> 8
   <212> PRT
   <213> Artificial
<220>
   <223> chemically synthesized
<400> 10
   Gly Arg Gly Cys Val Gln Asp Gly
   1               5
<210> 11
   <211> 8
   <212> PRT
   <213> Artificial
<220>
   <223> chemically synthesized
<400> 11
   Gly Arg Gly Leu Val Gln Pro Gly
   1               5
<210> 12
   <211> 8
   <212> PRT
   <213> Artificial
<220>
   <223> chemically synthesized
<400> 12
   Gly Arg Gly Leu Val Gln Asp Gly
   1               5
<210> 13
   <211> 8
   <212> PRT
   <213> Artificial
<220>
   <223> chemically synthesized
<400> 13
   Gly Gly Val Cys Val Gln Pro Gly
   1               5
<210> 14
   <211> 8
   <212> PRT
   <213> Artificial
<220>
   <223> chemically synthesized
<400> 14
   Gly Gly Val Cys Val Gln Asp Gly
   1               5
<210> 15
   <211> 8
   <212> PRT
   <213> Artificial
<220>
   <223> chemically synthesized
<400> 15
   Gly Gly Val Leu Val Gln Pro Gly
   1               5
<210> 16
   <211> 8
   <212> PRT
   <213> Artificial
<220>
   <223> chemically synthesized
<400> 16
   Gly Gly Val Leu Val Gln Asp Gly
   1               5
<210> 17
   <211> 8
   <212> PRT
   <213> Artificial
<220>
   <223> chemically synthesized
<400> 17
   Gly Gly Leu Cys Val Gln Pro Gly
   1               5
<210> 18
   <211> 8
   <212> PRT
   <213> Artificial
<220>
   <223> chemically synthesized
<400> 18
   Gly Gly Leu Cys Val Gln Asp Gly
   1               5
<210> 19
   <211> 8
   <212> PRT
   <213> Artificial
<220>
   <223> chemically synthesized
<400> 19
   Gly Gly Leu Leu Val Gln Pro Gly
   1               5
<210> 20
   <211> 8
   <212> PRT
   <213> Artificial
<220>
   <223> chemically synthesized
<400> 20
   Gly Gly Leu Leu Val Gln Asp Gly
   1               5
<210> 21
   <211> 8
   <212> PRT
   <213> Artificial
<220>
   <223> chemically synthesized
<400> 21
   Gly Gly Gly Cys Val Gln Pro Gly
   1               5
<210> 22
   <211> 8
   <212> PRT
   <213> Artificial
<220>
   <223> chemically synthesized
<400> 22
   Gly Gly Gly Cys Val Gln Asp Gly
   1               5
<210> 23
   <211> 8
   <212> PRT
   <213> Artificial
<220>
   <223> chemically synthesized
<400> 23
   Gly Gly Gly Leu Val Gln Pro Gly
   1               5
<210> 24
   <211> 8
   <212> PRT
   <213> Artificial
<220>
   <223> chemically synthesized
<400> 24
   Gly Gly Gly Leu Val Gln Asp Gly
   1               5

## Description

### FIELD OF THE INVENTION

The present invention relates to the fields of therapeutics. In particular the invention relates to treating asthma.

### BACKGROUND OF THE INVENTION

Mammalian epithelia contain structures referred to as zonula occludens (ZO) also referred to as tight junctions (TJs). These structures regulate the passage of materials through the epithelia by controlling access to the space between the epithelial cells (the paracellular pathway). To meet the many diverse physiological and pathological challenges to which epithelia are subjected, the tight junctions or zonula occludens must be capable of rapid, physiologic, reversible, transient, energy dependent, and coordinated responses that require the presence of a complex regulatory system. Examples of epithelia containing tight junctions include, but are not limited to, the intestines, (particularly the small intestine), the lungs, and the blood brain barrier.

In the absence of stimuli, the tight junctions are closed restricting access to the paracellular pathway. In the presence of stimuli, the tight junctions are reversibly opened. In U.S. Patent Nos. 5,945,510 and 5,948,629, novel mammalian proteins that function as the physiological modulator of mammalian tight junctions, have been identified and purified. These mammalian proteins, referred to as "zonulin," function as the physiological effector of mammalian tight junctions. Certain bacteria have been shown to have toxins that stimulate the opening of tight junctions. *Vibrio cholerae* infected with the filamentous bacteriophage CTXΦ, produces a toxin (zonula occludens toxin, ZOT) that has been shown to cause opening of tight junctions. It has been shown that 6 His-ΔG, an N-terminal deletion of ZOT in which the first 264 amino acids have been deleted and replaced with a six histidine purification tag, retains the ability to open tight junctions.

Intestinal tight junction dysfunction occurs in auto-immune diseases and in a variety of clinical conditions affecting the gastrointestinal tract, including food allergies, enteric infections, malabsorption syndromes such as celiac disease, and inflammatory bowel diseases. Healthy, mature gut mucosa with its intact tight junction serves as the main barrier to the passage of macromolecules. During the healthy state, small quantities of immunologically active proteins cross the gut host barrier. When the integrity of the tight junction system is compromised, as with prematurity or after exposure to radiation, chemotherapy, and/or toxins, a deleterious response to environmental antigens (including autoimmune diseases and food allergies) can occur.

Peptide antagonists of zonulin were described in U.S. Patent 6,458,925, which corresponds to WO 00/07609. Peptide antagonists of zonulin may bind to the ZOT receptor, yet not function to physiologically modulate the opening of mammalian tight junctions. The peptide antagonists competitively inhibit the binding of ZOT and zonulin to the ZOT receptor, thereby inhibiting the ability of ZOT and zonulin to physiologically modulate the opening of mammalian tight junctions.

Asthma is a chronic lung disorder that is marked by recurring episodes of airway obstruction (as from bronchospasm) manifested by labored breathing accompanied especially by wheezing and coughing and by a sense of constriction in the chest, and that is triggered by hyperreactivity to various stimuli (as allergens or rapid change in air temperature). In sensitized individuals, inhaled allergens (allergy triggers), such as pet dander, dust mites, cockroach allergens, molds, or pollens provoke a hyperimmune response characterized by recruitment of immune cells and production of IgE antibodies. Currently, asthma is generally treated using long term treatment with anti-inflammatories (e.g., glucocorticoids) and long-acting bronchodilators in combination with short term episodic treatment of acute attacks with short-acting bronchodilators. Glucocorticoids, the current gold standard treatment for allergic asthma, have been shown to act by suppressing the adaptive immune response (e.g., recruitment of inflammatory cells) while not suppressing innate immune response (e.g., epithelial barrier function and complement expression) (see Schleimer RP, Proc Am Thorac Soc. 2004;1(3):222-30. Glucocorticoids suppress inflammation but spare innate immune responses in airway epithelium.)

The barrier function of the epithelium is the first line of defense of the innate immune system. The epithelium secrets a variety of molecules (e.g., complement, collectins, lysozyme, and defensins) that lead to destruction of pathogens before they have an opportunity to penetrate. As discussed above, tight junctions play an integral role in maintaining and regulating the barrier function. Disruption of lung tight junction function has been implicated in the development of allergic sensitization and asthma. In order for an allergen to reach antigen-presenting cells and induce an adaptive immune response, such as in asthma, the allergen must cross the lung epithelium. It has been shown that dust mite allergen Der p1 causes disruption of lung tight junction structure and an increase in the permeability of lung epithelia. Wan, et al. J Clinical Investigation 104(1):123-133 (1999). It was suggested that the transepithelial movement of Der p1 may have been facilitated by the inherent proteolytic activity of Der p 1.

Reed and Kita (2004) states that PAR stimulation of epithelial cells opens tight junctions and that PAR-2 is increased on the epithelium of patients with asthma. However, they also state that it is not yet known which treatment is effective in reversing the effects of PARs. In addition, they also state that no specific PAR antagonists are available for treatment, although PAR antagonists under development include lapidated peptides and peptide-mimetic antagonists (J. Allergy Clin. Immunol. 2004;114:997-1008.)

US2005/0059593 states that the receptor for the peptide having the sequence of SEQ ID NO: 15, is present on nasal mucosa and on bovine pulmonary artery (BPA) endothelial cells. It does not disclose the presence of the receptor on or near the lung epithelium. From the examples disclosed in US2005/0059593 it is not clear if the peptide blocks tight junction opening by binding to PAR-2 or binding to a PAR-2 variant or homologue. Moreover, the authors of US2005/0059593 provide a summary of their results which discourage the skilled person from assuming that the target is PAR-2.

There remains a need in the art for methods and materials for the diagnosis and treatment of asthma. This need an others are met by the present invention.

### SUMMARY OF THE INVENTION

The present invention is as set out in the claims. Described herein are materials and methods for diagnosing asthma in a subject. A method of diagnosing asthma in a subject may comprise obtaining a sample from the subject and determining zonulin in the sample, wherein the presence of zonulin is predictive of asthma. Any suitable sample may be used, for example, a bronchoalveolar lavage (BAL) sample. Such methods may also include determining one or more chemokines in the sample, for example, determining one or more of TARC, MDC, and IP-10.

Also described herein are kits for diagnosing asthma. Such kits may comprise means for detecting zonulin. Such kits may also comprise means for detecting at least one cytokine. Means for detecting zonulin may comprise a first container containing a first antibody and a second container containing a second antibody. Kits may also comprise one or more compounds that may be used as control compounds to assess the activity of the reagents supplied in the kit. For example, to assess the activity of an antibody, the kits may comprise an antigen normally bound by the antibody. For example, a kit may comprise one or more containers containing ΔG fragment of zonula occludens toxin, ZOT, zonulin, TARC, MDC, or IP-10. Optionally, kits may comprise at least one antibody was raised against a protein comprising a fragment of zonula occludens toxin, for example, ΔG fragment of zonula occludens toxin. Kits may comprise antibodies raised against a protein comprising zonula occludens toxin. One or more antibodies used in kits of the invention may comprise one or more detectable moieties, e.g., biotin, fluorophores, chromophore, enzymes and the like.

Practice of some methods described herein may require the determination of zonulin. Determining zonulin includes both detecting the presence or absence of zonulin in a sample as well as measuring the concentration of zonulin in a sample. Optionally, the concentration of zonulin may be measured, or alternatively the presence or absence of zonulin may be assayed. Zonulin may be determined using any technique known to those of skill in the art. Determining zonulin may comprise contacting the sample with a first antibody that binds to zonulin under binding conditions, contacting the bound sample with a second antibody that binds zonulin under binding conditions, and detecting the presence of bound second antibody. Any first and second antibodies may used so long as they bind to both bind to zonulin with sufficient affinity to permit detection. Optionally, at least one antibody may be raised against a protein comprising a fragment of zonula occludens toxin. The first antibody may be raised against a protein comprising a fragment of zonula occludens toxin, for example, the ΔG fragment of zonula occludens toxin. Optionally, at least one antibody may be raised against a protein comprising zonula occludens toxin. Optionally, the second antibody may be raised against a protein comprising zonula occludens toxin. Typically, the second antibody may comprise a detectable moiety, for example, biotin, fluorophores, chromophore, enzymes and the like.

The present invention relates to compositions and methods for preventing, ameliorating and/or treating asthma. In one embodiment, the present invention provides a composition comprising a tight junction antagonist for use in a method of preventing, ameliorating and/or treating asthma in a subject in need thereof, comprising contacting lung epithelium of the subject with a composition comprising a tight junction antagonist, wherein the tight junction antagonist is a peptide comprising the amino acid sequence of SEQ ID NO: 15. In the method, the lung of the subject is contacted with the tight junction antagonist. For example, a composition comprising a tight junction antagonist may be applied to the lung of a subject with asthma or susceptible to developing asthma. In the method, the tight junction antagonist is a peptide comprising the amino acid sequence of SEQ ID NO:15. Compositions for use in methods of the invention may also comprise one or more therapeutic agents.

Also described herein are methods of monitoring the treatment of asthma in a subject. Such methods may comprise obtaining a first sample from the subject, determining zonulin level in the first sample, obtaining a second sample, determining zonulin level in the second sample, wherein a difference in zonulin level between the first sample and the second sample is indicative of a change in severity of asthma in the subject. The zonulin level in the second sample may be lower than the zonulin level in the first sample and the change in level may indicate a reduction in severity of one or more asthma symptoms. The zonulin level in the second sample may be higher than the zonulin level in the first sample and the change in level may indicate an increase in severity. Any suitable sample may be used so long as the presence of zonulin in the sample is related to the subject's asthma. For example, the samples may be bronchoalveolar lavage (BAL) samples and/or serum samples. Methods may further comprise determining one or more chemokines in the samples. Examples of cytokines that may be determined include, but are not limited to, one or more of TARC, MDC, and IP-10.

### BRIEF DESCRIPTION OF THE DRAWINGS

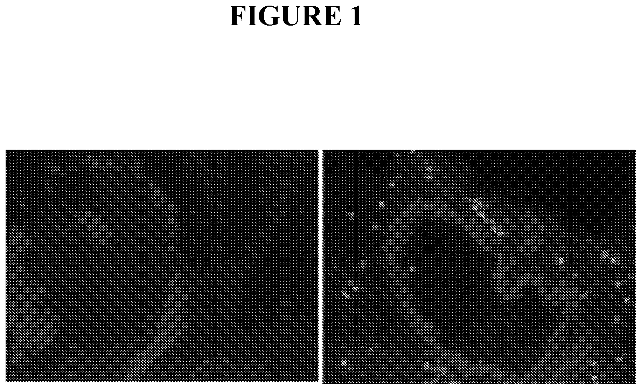

Figure 1 is a micrograph of an *in situ* immunoflourescence microscopy of mouse lung sections incubated with either FITC-FZI/0 (right panel) or FITC-FZI/1 (left panel). Clusters of immuno fluorescent FZI/0 particles were visualized at the level of distal airway tree, while no signal was detected in tissues incubated with FZI/1.

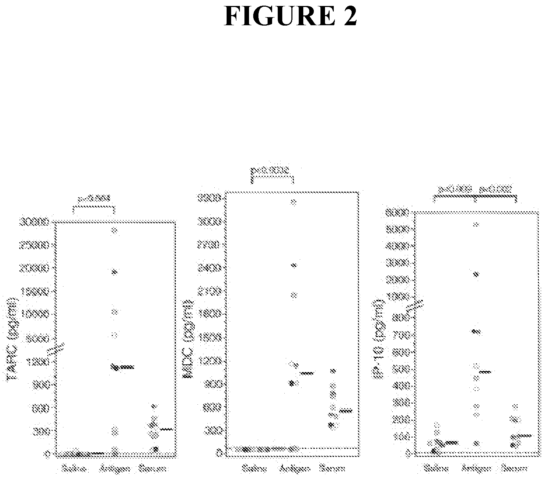

Figure 2 shows the results of assays of the levels of TARC, MDC, and IP-10 measured by commercial ELISAs in serum and in BAL fluids obtained 20 hours after segmental allergen challenge of atopic subjects with saline or allergen (n = 10; each colored dot represents a different subject). Short solid lines represent median values. Limits of detection are represented by dashed horizontal lines. P values are from the Mann-Whitney.

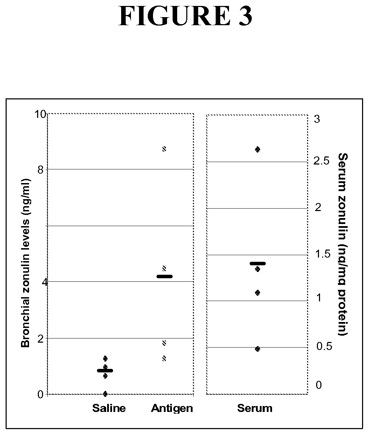

Figure 3 shows the results of assays of levels of zonulin measured by sandwich ELISA in serum and in BAL fluids obtained 20 hours after segmental allergen challenge of atopic subjects with saline or allergen (n = 4; each colored dot represents a different subject). Short solid lines represent median values.

### DETAILED DESCRIPTION OF THE INVENTION

As used herein a subject is any animal, e.g., mammal, upon which methods of the invention may be practiced and/or to which materials of the present invention may be administered. Subjects include, but are not limited to, humans.

As discussed above, described herein are materials and methods for diagnosing asthma. The present invention provides materials and methods for preventing, slowing the onset of, ameliorating and/or treating asthma in a subject in need thereof by, *inter alia,* administering to a subject in need of such preventing, slowing the onset of, ameliorating and/or treating, a pharmaceutically effective amount of a tight junction antagonist, wherein the tight junction antagonist is a peptide comprising the amino acid sequence of SEQ ID NO: 15. Antagonists suitable for use in the present invention bind to the zonula occludens toxin (ZOT) receptor, yet do not physiologically modulate the opening of mammalian tight junctions. The antagonists are peptides comprising the amino acid sequence of SEQ ID NO: 15. The term "antagonist" is defined as a compound that that prevents, inhibits, reduces or reverses the response triggered by an agonist (e.g. zonulin). In one embodiment, the present invention provides a composition as set out in the claims for treating asthma in a subject in need thereof by, *inter alia,* administering to a subject in need of such treating, a pharmaceutically effective amount of an antagonist of tight junction opening.

It has been shown that allergens can disrupt tight junctions in lung epithelium resulting in the introduction of the allergen to antigen presenting cells and that this might be an initial step in the development of asthma. Wan *et al. supra.* Without wishing to be bound by theory, the present invention provides evidence that transient disassembly of tight junctions occurs in asthma and may be a zonulin-mediated event. Accordingly, the transient disassembly of tight junctions can be reduced and/or prevented by the administration of an antagonist of tight junction opening, for example, an antagonist of zonulin. Using the materials and methods of the present invention it is possible to treat asthma by administering one or more compounds that prevent or reduce the transient disassembly of tight junctions in the lung epithelium. For example, zonulin is a normal physiological compound that enhances the permeability of anatomical barriers, for example, the lung epithelium, by mediating the opening of tight junctions. By administering a zonulin antagonist the permeability of the lung epithelium is maintained or decreased, thereby preventing, slowing the onset of, ameliorating and/or treating asthma.

### Antagonists of tight junction opening

As used herein, tight junction antagonists prevent, inhibit or reduce the opening of tight junctions, for example, the opening of tight junctions induced by a tight junction agonist. A tight junction antagonist may bind to the receptor that mediates tight junction agonist induced opening of tight junctions. As used herein an antagonist of zonulin is any compound that bind to the zonulin receptor and that prevents, inhibits, reduces or reverses the response triggered by zonulin. Antagonists of the invention are peptide antagonists comprising the amino acid sequence of SEQ ID NO: 15. Other examples of peptide antagonists include, peptides that comprise an amino acid sequence selected from the group consisting of
Gly Arg Val Cys Val Gln Pro Gly (SEQ ID NO:1),
Gly Arg Val Cys Val Gln Asp Gly (SEQ ID NO:2),
Gly Arg Val Leu Val Gln Pro Gly (SEQ ID NO:3),
Gly Arg Val Leu Val Gln Asp Gly (SEQ ID NO:4),
Gly Arg Leu Cys Val Gln Pro Gly (SEQ ID NO:5),
Gly Arg Leu Cys Val Gln Asp Gly (SEQ ID NO:6),
Gly Arg Leu Leu Val Gln Pro Gly (SEQ ID NO:7),
Gly Arg Leu Leu Val Gln Asp Gly (SEQ ID NO:8),
Gly Arg Gly Cys Val Gln Pro Gly (SEQ ID NO:9),
Gly Arg Gly Cys Val Gln Asp Gly (SEQ IDNO:10),
Gly Arg Gly Leu Val Gln Pro Gly (SEQ IDNO:11),
Gly Arg Gly Leu Val Gln Asp Gly (SEQ IDNO:12),
Gly Gly Val Cys Val Gln Pro Gly (SEQ ID NO:13),
Gly Gly Val Cys Val Gln Asp Gly (SEQ ID NO:14),
Gly Gly Val Leu Val Gln Asp Gly (SEQ ID NO:16),
Gly Gly Leu Cys Val Gln Pro Gly (SEQ ID NO:17),
Gly Gly Leu Cys Val Gln Asp Gly (SEQ ID NO:18),
Gly Gly Leu Leu Val Gln Pro Gly (SEQ ID NO:19),
Gly Gly Leu Leu Val Gln Asp Gly (SEQ ID NO:20),
Gly Gly Gly Cys Val Gln Pro Gly (SEQ ID NO:21),
Gly Gly Gly Cys Val Gln Asp Gly (SEQ ID NO:22),
Gly Gly Gly Leu Val Gln Pro Gly (SEQ ID NO:23), and
Gly Gly Gly Leu Val Gln Asp Gly (SEQ ID NO:24)

Any length of peptide may be used. Generally, the size of the peptide antagonist will range from about 6 to about 100, from about 6 to about 90, from about 6 to about 80, from about 6 to about 70, from about 6 to about 60, from about 6 to about 50, from about 6 to about 40, from about 6 to about 30, from about 6 to about 25, from about 6 to about 20, from about 6 to about 15, from about 6 to about 14, from about 6 to about 13, from about 6 to about 12, from about 6 to about 11, from about 6 to about 10, from about 6 to about 9, or from about 6 to about 8 amino acids in length. Peptide antagonists of the invention may be from about 8 to about 100, from about 8 to about 90, from about 8 to about 80, from about 8 to about 70, from about 8 to about 60, from about 8 to about 50, from about 8 to about 40, from about 8 to about 30, from about 8 to about 25, from about 8 to about 20, from about 8 to about 15, from about 8 to about 14, from about 8 to about 13, from about 8 to about 12, from about 8 to about 11, or from about 8 to about 10 amino acids in length. Peptide antagonists of the invention may be from about 10 to about 100, from about 10 to about 90, from about 10 to about 80, from about 10 to about 70, from about 10 to about 60, from about 10 to about 50, from about 10 to about 40, from about 10 to about 30, from about 10 to about 25, from about 10 to about 20, from about 10 to about 15, from about 10 to about 14, from about 10 to about 13, or from about 10 to about 12 amino acids in length. Peptide antagonists of the invention may be from about 12 to about 100, from about 12 to about 90, from about 12 to about 80, from about 12 to about 70, from about 12 to about 60, from about 12 to about 50, from about 12 to about 40, from about 12 to about 30, from about 12 to about 25, from about 12 to about 20, from about 12 to about 15, or from about 12 to about 14 amino acids in length. Peptide antagonists of the invention may be from about 15 to about 100, from about 15 to about 90, from about 15 to about 80, from about 15 to about 70, from about 15 to about 60, from about 15 to about 50, from about 15 to about 40, from about 15 to about 30, from about 15 to about 25, from about 15 to about 20, from about 19 to about 15, from about 15 to about 18, or from about 17 to about 15 amino acids in length.

The peptide antagonists can be chemically synthesized and purified using well-known techniques, such as described in High Performance Liquid Chromatography of Peptides and Proteins: Separation Analysis and Conformation, Eds. Mant et al., C.R.C. Press (1991), and a peptide synthesizer, such as Symphony (Protein Technologies, Inc); or by using recombinant DNA techniques, *i.e.,* where the nucleotide sequence encoding the peptide is inserted in an appropriate expression vector, *e.g.,* an *E. coli* or yeast expression vector, expressed in the respective host cell, and purified therefrom using well-known techniques.

### Therapeutic agents

Compositions of the invention may comprise one or more therapeutic agents. Any therapeutic agent that is useful in the treatment of asthma may be used in conjunction with an antagonist of tight junction opening in compositions of the invention. Examples of suitable therapeutic agents include, but are not limited to, medications that relieve asthma symptoms by relaxing muscles that have tightened around the airways. Other suitable therapeutic agents include medications that can prevent, reduce or reverse the swelling in the airways that causes asthma symptoms (e.g., anti-inflamatories). Other suitable therapeutic agents include long-acting bronchodilators that may be used together with anti-inflammatory medications. Examples of suitable medications include, but are not limited to, β-2-agonists, albuterol, metaproterenol sulfate, Combivent (a combination of a β-2-agonist and an anticholinergic), Duoneb (a combination of a β-2-agonist and an anticholinergic), Maxair, Tornalate, Ventolin, Xoponex, anticholinergics (such as ipratropium), anti-inflammatory drugs such as cromolyn and nedocromil, beclomethasone, budesonide (e.g., Pulmicort Turbuhaler), fluticasone (e.g., Flovent HFA), mometasone, corticosteroids, salmeterol, formoterol, Advair (a combination of a β-2-agonist and anti-inflammatory drug), Serevent, and Foradil.

### Formulations

Compositions of the invention are formulated for pulmonary delivery (e.g., may be pulmonary dosage forms). Typically such compositions may be provided as pharmaceutical aerosols, which may be solution aerosols and/or powder aerosols. Those of skill in the art are aware of many different methods and devices for the formation of pharmaceutical aerosols, for example, those disclosed by Sciarra and Sciarra, Aerosols, in Remington: The Science and Practice of Pharmacy, 20th Ed., Chapter 50, Gennaro et al. Eds., Lippincott, Williams and Wilkins Publishing Co., (2000).

Typically, compositions comprising a tight junction antagonist (peptide antagonist) comprise a pharmaceutically effective amount of the antagonist. The pharmaceutically effective amount of antagonist (e.g., peptide antagonist) employed may vary according to factors such as the disease state, age, sex, and weight of the individual. Dosage regimens may be adjusted to provide the optimum therapeutic response. For example, a single bolus may be administered, several divided doses may be administered over time or the dose may be proportionally reduced or increased as indicated by the exigencies of the therapeutic situation.

In one embodiment, the dosage forms are in the form of a powder aerosol (i.e, comprise particles). These are particularly suitable for use in inhalation delivery systems. Powders may comprise particles of any size suitable for administration to the lung.

Powder formulations may optionally contain at least one particulate pharmaceutically acceptable carrier known to those of skill in the art. Examples of suitable pharmaceutical carriers include, but are not limited to, saccharides, including monosaccharides, disaccharides, polysaccharides and sugar alcohols such as arabinose, glucose, fructose, ribose, mannose, sucrose, trehalose, lactose, maltose, starches, dextran, mannitol or sorbitol. In one embodiment, a powder formulation may comprise lactose as a carrier.

Powder formulations may be contained in any container known to those in the art. Containers may be capsules of, for example, gelatin or plastic, or in blisters (e.g. of aluminum or plastic), for use in a dry powder inhalation device. In some embodiments, the total weight of the formulation in the container may be from about 5 mg to about 50 mg. In other embodiments, powder formulations may be contained in a reservoir in a multi-dose dry powder inhalation device adapted to deliver a suitable amount per actuation.

Powder formulations typically comprise small particles. Suitable particles can be prepared using any means known in the art, for example, by grinding in an airjet mill, ball mill or vibrator mill, sieving, microprecipitation, spray-drying, lyophilisation or controlled crystallisation. Typically, particles will be about 10 microns or less in diameter. Particles for use in the compositions of the invention may have a diameter of from about 0.1 microns to about 10 microns, from about 0.1 microns to about 9 microns, from about 0.1 microns to about 8 microns, from about 0.1 microns to about 7 microns, from about 0.1 microns to about 6 microns, from about 0.1 microns to about 5 microns, from about 0.1 microns to about 4 microns, from about 0.1 microns to about 3 microns, from about 0.1 microns to about 2 microns, from about 0.1 microns to about 1 micron, from about 0.1 microns to about 0.5 microns, from about 1 micron to about 10 microns, from about 1 micron to about 9 microns, from about 1 micron to about 8 microns, from about 1 micron to about 7 microns, from about 1 micron to about 6 microns, from about 1 micron to about 5 microns, from about 1 micron to about 4 microns, from about 1 micron to about 3 microns, from about 1 micron to about 2 microns, from about 2 microns to about 10 microns, from about 2 microns to about 9 microns, from about 2 microns to about 8 microns, from about 2 microns to about 7 microns, from about 2 microns to about 6 microns, from about 2 microns to about 5 microns, from about 2 microns to about 4 microns, or from about 2 microns to about 3 microns. In some embodiments, particles for use in the invention may be about 1 micron, about 2 microns, about 3 microns, about 4 microns, about 5 microns, about 6 microns, about 7 microns, about 8 microns, about 9 microns, or about 10 microns in diameter.

In another embodiment, the dosage forms are in the form of a solution aerosol (i.e., comprise droplets). Solution aerosols may be prepared using any means known to those of skill in the art, for example, an aerosol vial provided with a valve adapted to deliver a metered dose (e.g., 10 µl to 100 µl, e.g. 25 µl to 50 µl) of the composition Where the inhalable form of the active ingredient is a nebulizable aqueous, organic or aqueous/organic dispersion, the inhalation device may be a nebulizer, for example a conventional pneumatic nebulizer such as an airjet nebulizer, or an ultrasonic nebulizer, which may contain, for example, from 1 to 50 ml, commonly 1 to 10 ml, of the dispersion; or a hand-held nebulizer which allows smaller nebulized volumes, e.g. 10 µl to 100 µl. Typically, droplets will be about 10 microns or less in diameter. Particles and/or droplets for use in the compositions of the invention may have a diameter of from about 0.1 microns to about 10 microns, from about 0.1 microns to about 9 microns, from about 0.1 microns to about 8 microns, from about 0.1 microns to about 7 microns, from about 0.1 microns to about 6 microns, from about 0.1 microns to about 5 microns, from about 0.1 microns to about 4 microns, from about 0.1 microns to about 3 microns, from about 0.1 microns to about 2 microns, from about 0.1 microns to about 1 micron, from about 0.1 microns to about 0.5 microns, from about 1 micron to about 10 microns, from about 1 micron to about 9 microns, from about 1 micron to about 8 microns, from about 1 micron to about 7 microns, from about 1 micron to about 6 microns, from about 1 micron to about 5 microns, from about 1 micron to about 4 microns, from about 1 micron to about 3 microns, from about 1 micron to about 2 microns, from about 2 microns to about 10 microns, from about 2 microns to about 9 microns, from about 2 microns to about 8 microns, from about 2 microns to about 7 microns, from about 2 microns to about 6 microns, from about 2 microns to about 5 microns, from about 2 microns to about 4 microns, or from about 2 microns to about 3 microns. In some embodiments, particles and/or droplets for use in the invention may be about 1 micron, about 2 microns, about 3 microns, about 4 microns, about 5 microns, about 6 microns, about 7 microns, about 8 microns, about 9 microns, or about 10 microns in diameter.

Compositions of the invention may comprise the tight junction antagonist at a level of from about 0.000001 wt% to about 50 wt%, from about 0.000001 wt% to about 45 wt%, from about 0.000001 wt% to about 40 wt%, from about 0.000001 wt% to about 35 wt%, from about 0.000001 wt% to about 30 wt%, from about 0.000001 wt% to about 25 wt%, from about 0.000001 wt% to about 20 wt%, from about 0.000001 wt% to about 15 wt%, from about 0.000001 wt% to about 10 wt%, from about 0.000001 wt% to about 5 wt%, from about 0.000001 wt% to about 2.5 wt%, from about 0.000001 wt% to about 1 wt%, from about 0.000001 wt% to about 0.1 wt%, from about 0.000001 wt% to about 0.01 wt%, from about 0.000001 wt% to about 0.001 wt%, from about 0.000001 wt% to about 0.0001 wt%, from about 0.000001 wt% to about 0.00005 wt%, from about 0.0001 wt% to about 50 wt%, from about 0.0001 wt% to about 45 wt%, from about 0.0001 wt% to about 40 wt%, from about 0.0001 wt% to about 35 wt%, from about 0.0001 wt% to about 30 wt%, from about 0.0001 wt% to about 25 wt%, from about 0.0001 wt% to about 20 wt%, from about 0.0001 wt% to about 15 wt%, from about 0.0001 wt% to about 10 wt%, from about 0.0001 wt% to about 5 wt%, from about 0.0001 wt% to about 2.5 wt%, from about 0.0001 wt% to about 1 wt%, from about 0.0001 wt% to about 0.1 wt%, from about 0.0001 wt% to about 0.01 wt%, from about 0.0001 wt% to about 0.001 wt%, from about 0.0001 wt% to about 0.0005 wt%, from about 0.1 wt% to about 50 wt%, from about 0.1 wt% to about 45 wt%, from about 0.1 wt% to about 40 wt%, from about 0.1 wt% to about 35 wt%, from about 0.1 wt% to about 30 wt%, from about 0.1 wt% to about 25 wt%, from about 0.1 wt% to about 20 wt%, from about 0.1 wt% to about 15 wt%, from about 0.1 wt% to about 10 wt%, from about 0.1 wt% to about 5 wt%, from about 0.1 wt% to about 2.5 wt%, from about 0.1 wt% to about 1 wt%, from about 0.1 wt% to about 0.5 wt%, from about 0.1 wt% to about 0.2 wt%, from about 1 wt% to about 50 wt%, from about 1 wt% to about 45 wt%, from about 1 wt% to about 40 wt%, from about 1 wt% to about 35 wt%, from about 1 wt% to about 30 wt%, from about 1 wt% to about 25 wt%, from about 1 wt% to about 20 wt%, from about 1 wt% to about 15 wt%, from about 1 wt% to about 10 wt%, from about 1 wt% to about 5 wt%, from about 1 wt% to about 2.5 wt%, from about 5 wt% to about 50 wt%, from about 5 wt% to about 45 wt%, from about 5 wt% to about 40 wt%, from about 5 wt% to about 35 wt%, from about 5 wt% to about 30 wt%, from about 5 wt% to about 25 wt%, from about 5 wt% to about 20 wt%, from about 5 wt% to about 15 wt%, from about 5 wt% to about 10 wt%, from about 5 wt% to about 9 wt%, from about 5 wt% to about 8 wt%, from about 5 wt% to about 7 wt%, or from about 5 wt% to about 6 wt% of the total weight of the composition. Compositions of the invention may comprise one or more tight junction antagonists at a level of about 0.00001 wt%, about 0.00005 wt%, about 0.0001 wt%, about 0.0005 wt%, about 0.001 wt%, about 0.005 wt%, about 0.01 wt%, about 0.05 wt%, about 0.1 wt%, about 0.5 wt%, about 1 wt%, about 5 wt%, about 10 wt%, about 15 wt%, about 20 wt%, about 25 wt%, about 30 wt%, about 35 wt%, about 40 wt%, about 45 wt%, or about 50 wt% based on the total weight of the composition.

Compositions of the invention may comprise one or more therapeutic agents at a concentration sufficient to cause the desired biological response (e.g., at a pharmaceutically effective concentration). Compositions of the invention may comprise one or more therapeutic agents at a level of from about 0.1 wt% to about 50 wt%, from about 0.001 wt% to about 45 wt%, from about 0.001 wt% to about 40 wt%, from about 0.001 wt% to about 35 wt%, from about 0.001 wt% to about 30 wt%, from about 0.001 wt% to about 25 wt%, from about 0.001 wt% to about 20 wt%, from about 0.001 wt% to about 15 wt%, from about 0.001 wt% to about 10 wt%, from about 0.001 wt% to about 5 wt%, from about 0.001 wt% to about 2.5 wt%, from about 0.001 wt% to about 1 wt%, from about 0.001 wt% to about 0.5 wt%, from about 0.001 wt% to about 0.2 wt%, 0.01 wt% to about 50 wt%, from about 0.01 wt% to about 45 wt%, from about 0.01 wt% to about 40 wt%, from about 0.01 wt% to about 35 wt%, from about 0.01 wt% to about 30 wt%, from about 0.01 wt% to about 25 wt%, from about 0.01 wt% to about 20 wt%, from about 0.01 wt% to about 15 wt%, from about 0.01 wt% to about 10 wt%, from about 0.01 wt% to about 5 wt%, from about 0.01 wt% to about 2.5 wt%, from about 0.01 wt% to about 1 wt%, from about 0.01 wt% to about 0.5 wt%, from about 0.01 wt% to about 0.2 wt%, 0.1 wt% to about 50 wt%, from about 0.1 wt% to about 45 wt%, from about 0.1 wt% to about 40 wt%, from about 0.1 wt% to about 35 wt%, from about 0.1 wt% to about 30 wt%, from about 0.1 wt% to about 25 wt%, from about 0.1 wt% to about 20 wt%, from about 0.1 wt% to about 15 wt%, from about 0.1 wt% to about 10 wt%, from about 0.1 wt% to about 5 wt%, from about 0.1 wt% to about 2.5 wt%, from about 0.1 wt% to about 1 wt%, from about 0.1 wt% to about 0.5 wt%, from about 0.1 wt% to about 0.2 wt%, from about 1 wt% to about 50 wt%, from about 1 wt% to about 45 wt%, from about 1 wt% to about 40 wt%, from about 1 wt% to about 35 wt%, from about 1 wt% to about 30 wt%, from about 1 wt% to about 25 wt%, from about 1 wt% to about 20 wt%, from about 1 wt% to about 15 wt%, from about 1 wt% to about 10 wt%, from about 1 wt% to about 5 wt%, from about 1 wt% to about 2.5 wt%, from about 5 wt% to about 50 wt%, from about 5 wt% to about 45 wt%, from about 5 wt% to about 40 wt%, from about 5 wt% to about 35 wt%, from about 5 wt% to about 30 wt%, from about 5 wt% to about 25 wt%, from about 5 wt% to about 20 wt%, from about 5 wt% to about 15 wt%, from about 5 wt% to about 10 wt%, from about 5 wt% to about 9 wt%, from about 5 wt% to about 8 wt%, from about 5 wt% to about 7 wt%, or from about 5 wt% to about 6 wt% of the total weight of the composition. Compositions of the invention may comprise one or more therapeutic agents at a level of about 0.1 wt%, about 1 wt%, about 5 wt%, about 10 wt%, about 15 wt%, about 20 wt%, about 25 wt%, about 30 wt%, about 35 wt%, about 40 wt%, about 45 wt%, or about 50 wt% based on the total weight of the composition.

Compositions of the invention may comprise one or more pharmaceutically acceptable excipients at a level of from about 0.1 wt% to about 50 wt%, from about 0.1 wt% to about 45 wt%, from about 0.1 wt% to about 40 wt%, from about 0.1 wt% to about 35 wt%, from about 0.1 wt% to about 30 wt%, from about 0.1 wt% to about 25 wt%, from about 0.1 wt% to about 20 wt%, from about 0.1 wt% to about 15 wt%, from about 0.1 wt% to about 10 wt%, from about 0.1 wt% to about 5 wt%, from about 0.1 wt% to about 2.5 wt%, from about 0.1 wt% to about 1 wt%, from about 0.1 wt% to about 0.5 wt%, from about 0.1 wt% to about 0.2 wt%, from about 1 wt% to about 50 wt%, from about 1 wt% to about 45 wt%, from about 1 wt% to about 40 wt%, from about 1 wt% to about 35 wt%, from about 1 wt% to about 30 wt%, from about 1 wt% to about 25 wt%, from about 1 wt% to about 20 wt%, from about 1 wt% to about 15 wt%, from about 1 wt% to about 10 wt%, from about 1 wt% to about 5 wt%, from about 1 wt% to about 2.5 wt%, from about 5 wt% to about 50 wt%, from about 5 wt% to about 45 wt%, from about 5 wt% to about 40 wt%, from about 5 wt% to about 35 wt%, from about 5 wt% to about 30 wt%, from about 5 wt% to about 25 wt%, from about 5 wt% to about 20 wt%, from about 5 wt% to about 15 wt%, from about 5 wt% to about 10 wt%, from about 5 wt% to about 9 wt%, from about 5 wt% to about 8 wt%, from about 5 wt% to about 7 wt%, or from about 5 wt% to about 6 wt% of the total weight of the composition. Compositions of the invention may comprise one or more pharmaceutically acceptable excipients at a level of about 0.1 wt%, about 1 wt%, about 5 wt%, about 10 wt%, about 15 wt%, about 20 wt%, about 25 wt%, about 30 wt%, about 35 wt%, about 40 wt%, about 45 wt%, or about 50 wt% based on the total weight of the composition.

Compositions of the invention may comprise one or more pharmaceutically-acceptable carriers. As used herein "pharmaceutically-acceptable carrier" includes any and all solvents, dispersion media, coatings, antibacterial and antifungal agents, isotonic and absorption delaying agents, and the like that are physiologically compatible. Pharmaceutically-acceptable carriers include, but are not limited to, sterile aqueous solutions or dispersions and sterile powders for the extemporaneous preparation of sterile injectable solutions or dispersion. The use of such media and agents for pharmaceutically active substances is well known in the art. Except insofar as any conventional media or agent is incompatible with the active compound, use thereof in the pharmaceutical compositions of the invention is contemplated. Supplementary active compounds can also be incorporated into the compositions.

Compositions of the invention may be formulated as a unit dose. A suitable unit dose of a peptide antagonist of the invention may be from about 20 µg to about 200 mg, from about 50 µg to about 200 mg, from about 100 µg to about 200 mg, from about 250 µg to about 200 mg, from about 500 µg to about 200 mg, from about 1 mg to about 200 mg, from about 2.5 mg to about 200 mg, from about 5 mg to about 200 mg, from about 10 mg to about 200 mg, from about 25 mg to about 200 mg, from about 50 mg to about 200 mg, or from about 100 mg to about 200 mg. For example, a suitable unit dose of peptide SEQ ID NO:15 may be from about 50 µg to about 50 mg. The precise amount of a unit dose will depend on the method of administration.

### Methods of use

The pharmaceutical compositions of the invention are used for treating asthma. In one embodiment, the present invention provides a composition for use in method of treating asthma as defined in the claims by administering a composition comprising one or more tight junction antagonists and one or more therapeutic agents. Pharmaceutical compositions of the invention may be used to prevent asthma and/or to reduce the frequency and/or severity of asthmatic attacks.

In some embodiments, compositions of the invention may be given repeatedly over a protracted period, i.e., may be chronically administered. Typically, compositions may be administered one or more times each day in an amount suitable to prevent or reduce the likelihood of an asthma attack. Such pharmaceutical compositions may be administered chronically, for example, one or more times daily over a plurality of days. An amount suitable to prevent or reduce the likelihood of an asthma attack may be a unit dose as described above.

In some embodiments, pharmaceutical compositions of the invention may be used to treat acute asthmatic attacks. Typically, embodiments of this type will require administration of the pharmaceutical compositions of the invention to a subject undergoing an asthmatic attack in an amount suitable to reduce the severity of the attack. One or more administration may be used.

A composition according to the present invention may be pre-mixed prior to administration, or can be formed *in vivo* when two or more components (e.g., a tight junction antagonist and a therapeutic agent) are administered within 24 hours of each other. When administered separately, the components may be administered in either order (e.g. tight junction antagonist first followed by therapeutic agent or therapeutic agent first followed by tight junction antagonist). The components can be administered within a time span of about 12 hours, about 8 hours, about 4 hours, about 2 hours, about 1 hour, about 0.5 hour, about 0.25 hour, about 0.1 hour, about 1 minute, about 0.5 minute, or about 0.1 minute.

Administration of the compositions described above, e.g., compositions comprising one or more tight junction antagonists and optionally comprising one or more therapeutic agents, may be by inhalation. For example, one or more tight junction antagonists and one or more therapeutic agents or a mixture thereof, may be in inhalable form. An example of an inhalable form is an atomizable composition such as an aerosol comprising the tight junction antagonist, either alone or in combination with one or more therapeutic agents, in solution or dispersion in a propellant, or a nebulizable composition comprising a solution or dispersion of the active ingredient in an aqueous, organic or aqueous/organic medium. For example, the inhalable form of the compositions of the invention may be an aerosol comprising a mixture of one or more tight junction antagonists and one or more therapeutic agents in solution or dispersion in a propellant, or a combination of an aerosol containing one or more tight junction antagonists in solution or dispersion in a propellant with an aerosol containing one or more therapeutic agents in solution or dispersion in a propellant. In another example, the inhalable form of the compositions of the invention my be a nebulizable composition comprising a dispersion of one or more tight junction antagonists and one or more therapeutic agents in an aqueous, organic or aqueous/organic medium, or a combination of a dispersion of one or more tight junction antagonists with a dispersion of one or more therapeutic agents in such a medium.

The following examples are provided for illustrative purposes only, and are in no way intended to limit the scope of the present invention.

### EXAMPLE 1

It has been previously demonstrated that the zonulin pathway is operative in the respiratory tract and can be specifically activated for antigen delivery strategies (see Marinaro M, Di Tommaso A, Uzzau S, Fasano A, De Magistris MT. Infect Immun. 1999 Mar;67(3):1287-91.Zonula occludens toxin is a powerful mucosal adjuvant for intranasally delivered antigens and United States Patent Application 20060165722).

*In situ* immunofluorescence microscopy was used to establish the distribution of the zonulin receptor within the respiratory tract. Lung tissue sections (4 µm) made from frozen blocks were placed immediately on plain uncoated slides and incubated with either FITC-labeled FZI/0 (the zonulin synthetic peptide inhibitor that specifically binds to the zonulin receptor described in U.S. patent no. 6,458,925, Gly-Gly-Val-Leu-Val-Gln-Pro-Gly SEQ ID NO: 15) or FITC-labeled FZI/1 (a scrambled peptide described in U.S. patent no. 6,458,925, Val-Gly-Val-Leu-Gly-Arg-Pro-Gly SEQ ID NO: 25) The results are shown in Figure 1. Cluster of immunofluorescence FZI/0 particles were visualized at the interface between endothelial (stained in red, Fig. 1) and epithelial layers, while no signal was detected in FZI/1-exposed tissues. These data suggest that zonulin receptors are present throughout the respiratory tract.

Human studies. Four subjects with both allergic rhinitis and asthma underwent segmental challenge with saline and antigen followed by bronchoalveolar lavage (BAL) as described by Bochner et al. (Bochner BS, Hudson SA, Xiao HQ, Liu MC. J Allergy Clin Immunol. 2003 Nov;112(5):930-4. Release of both CCR4-active and CXCR3-active chemokines during human allergic pulmonary late-phase reactions.) Briefly, for saline challenge, 5 mL of saline was instilled into 1 lung segment. For antigen challenge, 5 mL of low endotoxin ragweed or dust mite (*Dermatophagoides pteronyssinus*) antigen (Greer Laboratories, Lenoir, NC) at a concentration of 100 PNU/mL was instilled into another segment of the opposite lung. A second bronchoscopy was then performed 20 hours later with BAL to assess zonulin release and the inflammatory responses following both saline and allergen challenges. Blood was also obtained at the time of the second bronchoscopy. Cells were removed by centrifugation, and serum and BAL fluids were frozen at -80°C and analyzed at a later date (29).

As expected, allergen challenge induced a vigorous inflammatory cellular response (REF). Shown in Fig 2 are the ELISA results for the chemokines TARC (Thymus and activation-regulated chemokine), MDC (macrophage derived chemokine), and IP-10 (interferon-gamma-inducible protein 10) using BAL fluids and serum obtained 20 hours after segmental challenge. TARC and MDC levels were below the limit of detection in most of the samples from saline sites, but were detected in most BAL fluids from antigen-challenged sites. The same pattern was seen for IP-10, except that levels were detectable in BAL fluids at every saline site (median, 66 pg/mL). In comparing levels at antigen-challenged sites, TARC, MDC, and IP-10 were significantly increased compared with those at saline sites. Interestingly, the release of chemokines in the airway lumen was paralleled by zonulin release that resulted six-fold higher in segments challenged with antigen as compared to those challenged with saline (Fig 3, p<0.05).

## Claims

1. A composition comprising a tight junction antagonist, for use in a method of treatment of asthma in a subject in need thereof, said treatment comprising contacting the lung epithelium of the subject with the composition, wherein the tight junction antagonist is a peptide comprising the amino acid sequence of SEQ ID NO: 15.

2. The composition for use according to claim 1, wherein the composition further comprises a therapeutic agent selected from a muscle relaxer, a bronchodilator, and an anti-inflammatory.

3. The composition for use according to claim 2, wherein the therapeutic agent is selected from a β-2-agonist, albuterol, metaproterenol sulfate, Combivent, Duoneb, Maxair, Tornalate, Ventolin, Xoponex, an anticholinergic, ipratropium, cromolyn, nedocromil, beclomethasone, budesonide, fluticasone, mometasone, corticosteroids, salmeterol, formoterol, Advair, Serevent, and Foradil.

4. The composition for use according to claim 1, wherein the peptide is from 8 to about 14 amino acids in length.

5. The composition for use according to any one of claims 1 to 4, wherein the composition is provided as a solution aerosol or powder aerosol.

## Patentansprüche

1. Zusammensetzung, umfassend einen Tight-junction-Antagonisten zur Verwendung in einem Verfahren zur Behandlung von Asthma bei einem bedürftigen Individuum, wobei die Behandlung das Kontaktieren des Lungenepithels des Individuums mit der Zusammensetzung umfasst, wobei der Tight-junction-Antagonist ein Peptid ist, das die Aminosäuresequenz von SEQ ID NO: 15 umfasst.

2. Zusammensetzung zur Verwendung nach Anspruch 1, wobei die Zusammensetzung weiters ein Therapeutikum umfasst, das aus einem Muskelrelaxans, einem Bronchodilatator und einem Entzündungshemmer ausgewählt ist.

3. Zusammensetzung zur Verwendung nach Anspruch 2, wobei das Therapeutikum aus einem β-2-Agonisten, Albuterol, Metaproterenolsulfat, Combivent, Duoneb, Maxair, Tornalat, Ventolin, Xoponex, einem Anticholinergikum, Ipratropium, Cromolyn, Nedocromil, Beclomethason, Budesonid, Fluticason, Mometason, Corticosteroiden, Salmeterol, Formoterol, Advair, Serevent und Foradil ausgewählt ist.

4. Zusammensetzung zur Verwendung nach Anspruch 1, wobei das Peptid eine Länge von 8 bis etwa 14 Aminosäuren aufweist.

5. Zusammensetzung zur Verwendung nach einem der Ansprüche 1 bis 4, wobei die Zusammensetzung als Aerosollösung oder Aerosolpulver bereitgestellt ist.

## Revendications

1. Composition comprenant un antagoniste de jonction serrée, destinée à être utilisée dans une méthode de traitement de l'asthme chez un sujet qui en a besoin, ledit traitement comprenant la mise en contact de l'épithélium pulmonaire du sujet avec la composition, dans laquelle l'antagoniste de jonction serrée est un peptide comprenant la séquence d'acides aminés de SEQ ID NO : 15.

2. Composition à utiliser selon la revendication 1, dans laquelle la composition comprend en outre un agent thérapeutique choisi parmi un relaxant musculaire, un bronchodilatateur et un anti-inflammatoire.

3. Composition à utiliser selon la revendication 2, dans laquelle l'agent thérapeutique est choisi parmi un β-2-agoniste, l'albutérol, le sulfate de métaprotérénol, le Combivent, le Duoneb, le Maxair, le Tomalate, la Ventoline, le Xoponex, un anticholinergique, l'Ipratropium, le Cromolyn, le nédocromil, la béclométhasone, le budésonide, la fluticasone, la mométasone, les corticostéroïdes, le salmétérol, le formotérol, l'Advair, le Serevent et le Foradil.

4. Composition à utiliser selon la revendication 1, dans laquelle le peptide a une longueur de 8 à environ 14 acides aminés.

5. Composition à utiliser selon l'une quelconque des revendications 1 à 4, dans laquelle la composition est fournie sous forme d'aérosol en solution ou d'aérosol en poudre.
